# EUROPEAN PATENT APPLICATION

(11) **EP 4 837 920 A1**
(43) Date of publication of application: **11.11.2026**
(21) Application number: 25741479.7
(22) Date of filing: 14.01.2025
(51) Int. Cl.: C07K 16/28, C12N 15/13, A61K 39/395, A61P 37/02, G01N 33/577, G01N 33/68

(54) **ANTI-CD132 ANTIBODIES, AND USE THEREOF**

(30) Priority: 19.01.2024 CN 202410081646; 19.01.2024 CN 202411192750
(71) Applicant: Institute of Dermatology, Chinese Academy of Medical Sciences and Peking Union Medical College, Nanjing, Jiangsu 210042 (CN)
(72) Inventor: LU, Qianjin, Nanjing, Jiangsu 210042 (CN); YIN, Huiqi, Nanjing, Jiangsu 210042 (CN); FENG, Xiwei, Nanjing, Jiangsu 210042 (CN); LI, Liming, Nanjing, Jiangsu 210042 (CN); ZHAO, Junpeng, Nanjing, Jiangsu 210042 (CN); FAN, Xinyu, Nanjing, Jiangsu 210042 (CN)
(74) Representative: HGF
(86) International application number: PCT/CN2025/072214
(87) International publication number: WO 2025/152918

(57) **Abstract**

The invention belongs to the field of biomedicine, and particularly relates to a humanized anti-human CD132 monoclonal antibody and a use thereof. According to the invention, based on monoclonal chimeric antibodies 2D4 and 5H10 previously obtained in the laboratory, humanized transformation is performed on a target antibody, so as to screen and obtain high-affinity humanized monoclonal antibodies h2D4H4K12 and/or h5H10H6K4 targeting human CD132, so that, without attenuating antibody activities, immunogenicities of parental chimeric antibodies are reduced, thereby reducing the risk of potential immune responses to the antibodies in patients receiving the antibodies. The antibodies have promising application prospects in therapeutic drugs for prevention, neutralization or treatment of autoimmune diseases related to IL-4, IL-7, IL-9, IL-15 and/or IL-21 cytokines (such as systemic lupus erythematosus, rheumatoid arthritis, vitiligo, psoriasis, alopecia areata, inflammatory bowel disease, systemic sclerosis, graft-versus-host disease and aplastic anemia).

## Description

### TECHNICAL FIELD

The invention belongs to the field of biomedicine, and particularly relates to an anti-CD132 antibody and a use thereof.

### BACKGROUND

CD132 (IL-2Ry) is a γ subunit of an interleukin-2 (IL-2) receptor, and the subunit is simultaneously a y subunit of receptor complexes for 6 cytokines: IL-2, IL-4, IL-7, IL-9, IL-15 and IL-21. Therefore, the subunit is also referred to as γc (common y chain). Systemic lupus erythematosus (SLE) is a chronic autoimmune disease affecting multiple organs and systems. The IL-4, IL-7, IL-9, IL-15 and IL-21 among γ-chain family cytokines are significantly upregulated in immune cells of SLE, and these γ-chain cytokines play key regulatory roles in survival, proliferation and differentiation of various lymphocytes. Given that the SLE is a complex disease involving multiple cells and factors together, and critical regulatory functions of the γ-chain cytokines in various lymphocytes are closely associated with the pathogenesis and progression of the SLE, intervention of functional activity of the common subunit CD132 of the γ-chain cytokine receptors may become a promising therapeutic method for the SLE.

Furthermore, 6 upstream γ-chain cytokines of the CD132 also play pivotal roles in various other inflammatory diseases, wherein blockade of an IL-21 pathway has been extensively studied and applied in treatment of the autoimmune disease. The IL-21 acts directly on T cells and B cells to promote T cell activation, B cell proliferation and differentiation, and antibody production. Therefore, the blockade of the IL-21 exerts a therapeutic effect in multiple autoimmune diseases caused by dysfunction of T and B cells. For example, in rheumatoid arthritis (RA), the IL-21 contributes to the development of destructive arthritis, and inhibition of the IL-21 signaling pathway exhibits a therapeutic effect in treatment of the RA, which can alleviate joint inflammation and improve joint function [1]. In addition, inhibition of the IL-21 and the IL-7 exhibits a therapeutic effect in inflammatory bowel disease (IBD) [2, 3]. Multiple literatures have indicated that an IL-15 signaling pathway plays a key role in the development and progression of vitiligo, and blockade of the IL-15 signaling pathway can achieve long-term alleviation of vitiligo symptoms [4]. Immune cells of some patients with alopecia areata (AA) may exhibit abnormal expression of an IL-7 receptor (IL-7R), and blockade of the IL-7 may prevent disease progression of mice with alopecia areata, and even reverse early-stage alopecia areata [5]. In a systemic sclerosis model, the IL-21 drives inflammation and fibrosis in skin and lungs [6]. IL-15-induced bone marrow CD8+ Trm cells are involved in destruction of HSPC in human severe aplastic anemia, and the IL-21 also contributes to disease progression of the aplastic anemia [7, 8]. In addition, all the above autoimmune diseases arise from aberrant activation of the T cells, B cells or NK cells, which mistakenly attack autologous cells.

Moreover, the CD132 is the common subunit of the IL-4, IL-7, IL-9, IL-15 and IL-21 cytokine receptors, and the CD132 is also a key regulatory factor for functions of the T cells, B cells or NK cells. Therefore, targeting the CD132 may be a novel therapeutic direction for patients suffering from various autoimmune diseases, including systemic lupus erythematosus, rheumatoid arthritis, vitiligo, psoriasis, alopecia areata, inflammatory bowel disease, systemic sclerosis, graft-versus-host disease, aplastic anemia, and the like.
[1] Young D.A, et al. Blockade of the interleukin-21/interleukin-21 receptor pathway ameliorates disease in animal models of rheumatoid arthritis[J].
[2] Fina D, et al. Regulation of gut inflammation and th17 cell response by interleukin-21[J].
[3] Belarif L, et al. IL-7 receptor influences anti-TNF responsiveness and T cell gut homing in inflammatory bowel disease[J].
[4] Richmond, J.M, et al. Antibody blockade of IL-15 signaling has the potential to durably reverse vitiligo[J].
[5] Dai Z, et al. Blockade of IL-7 signaling suppresses inflammatory responses and reverses alopecia areata in C3H/HeJ mice[J].
[6] Um, I.G, et al. IL-21 drives skin and lung inflammation and fibrosis in a model for systemic sclerosis[J].
[7] Zhang, J, et al. Involvement of interleukin-21 in the pathophysiology of aplastic anemia[J].
[8] Long, J, et al. Bone marrow CD8+ Trm cells induced by IL-15 and CD16+ monocytes contribute to HSPC destruction in human severe aplastic anemia[J].

### SUMMARY

The technical problem to be solved by the present invention is to provide a humanized anti-human CD132 monoclonal antibody aiming at the defects in the prior art.

In order to solve the above technical problem, the following technical solution is used in the present invention.

In a first aspect, the present invention provides an antibody.

In one aspect, the present invention provides an antibody capable of specifically binding to CD132 or an antigen-binding fragment thereof.

In some embodiments, the antibody or the antigen-binding fragment thereof includes the following complementarity-determining regions (CDRs):
(a) CDR-H1, CDR-H2 and CDR-H3 contained in a heavy chain variable region (VH) as shown in SEQ ID NO. 1; and CDR-L1, CDR-L2 and CDR-L3 contained in a light chain variable region (VL) as shown in SEQ ID NO. 22;
(b) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 11;
(c) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 16;
(d) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 19;
(e) CDR-H1, CDR-H2 and CDR-H3 contained in a heavy chain variable region (VH) as shown in SEQ ID NO. 6; and CDR-L1, CDR-L2 and CDR-L3 contained in a light chain variable region (VL) as shown in SEQ ID NO. 24; and
(f) the CDR-H1, CDR-H2 and CDR-H3 contained in the heavy chain variable region (VH) as shown in SEQ ID NO. 6; and CDR-L1, CDR-L2 and CDR-L3 contained in a light chain variable region (VL) as shown in SEQ ID NO. 29.

The CDRs are defined according to a Kabat, IMGT, Chothia or AbM numbering system.

In some embodiments, the VH and/or the VL of the antibody or the antigen-binding fragment thereof include framework regions (FRs) derived from a human or mouse immunoglobulin.

In some embodiments, the antibody or the antigen-binding fragment thereof binds to human CD132.

In some embodiments, the antibody or the antigen-binding fragment thereof includes:
the following heavy chain variable region (VH) and light chain variable region (VL):
   (a) a heavy chain variable region (VH) containing the following 3 CDRs: CDR-H1 having a sequence as shown in SEQ ID NO. 3, CDR-H2 having a sequence as shown in SEQ ID NO. 4 and CDR-H3 having a sequence as shown in SEQ ID NO. 5; and,
a light chain variable region (VL) containing the following 3 CDRs: CDR-L1 having a sequence as shown in SEQ ID NO. 18, CDR-L2 having a sequence as shown in SEQ ID NO. 14 and CDR-L3 having a sequence as shown in SEQ ID NO. 15;
   (b) the heavy chain variable region (VH) containing the following 3 CDRs: the CDR-H1 having the sequence as shown in SEQ ID NO. 3, the CDR-H2 having the sequence as shown in SEQ ID NO. 4 and the CDR-H3 having the sequence as shown in SEQ ID NO. 5; and,
   a light chain variable region (VL) containing the following 3 CDRs: CDR-L1 having a sequence as shown in SEQ ID NO. 13, the CDR-L2 having the sequence as shown in SEQ ID NO. 14 and the CDR-L3 having the sequence as shown in SEQ ID NO. 15;
   (c) the heavy chain variable region (VH) containing the following 3 CDRs: the CDR-H1 having the sequence as shown in SEQ ID NO. 3, the CDR-H2 having the sequence as shown in SEQ ID NO. 4 and the CDR-H3 having the sequence as shown in SEQ ID NO. 5; and,
   a light chain variable region (VL) containing the following 3 CDRs: CDR-L1 having a sequence as shown in SEQ ID NO. 21, the CDR-L2 having the sequence as shown in SEQ ID NO. 14 and the CDR-L3 having the sequence as shown in SEQ ID NO. 15;
   (d) a heavy chain variable region (VH) containing the following 3 CDRs: CDR-H1 having a sequence as shown in SEQ ID NO. 8, CDR-H2 having a sequence as shown in SEQ ID NO. 9 and CDR-H3 having a sequence as shown in SEQ ID NO. 10; and,
   a light chain variable region (VL) containing the following 3 CDRs: CDR-L1 having a sequence as shown in SEQ ID NO. 26, CDR-L2 having a sequence as shown in SEQ ID NO. 27 and CDR-L3 having a sequence as shown in SEQ ID NO. 28; and
   (e) the heavy chain variable region (VH) containing the following 3 CDRs: the CDR-H1 having the sequence as shown in SEQ ID NO. 8, the CDR-H2 having the sequence as shown in SEQ ID NO. 9 and the CDR-H3 having the sequence as shown in SEQ ID NO. 10; and,
   a light chain variable region (VL) containing the following 3 CDRs: the CDR-L1 having the sequence as shown in SEQ ID NO. 26, the CDR-L2 having the sequence as shown in SEQ ID NO. 27 and CDR-L3 having a sequence as shown in SEQ ID NO. 31.

In some embodiments, the antibody or the antigen-binding fragment thereof includes the heavy chain variable region (VH) and the light chain variable region (VL) selected from any one of the following groups:
(g) the VH having the sequence as shown in SEQ ID NO. 1 and the VL having the sequence as shown in SEQ ID NO. 22;
(h) the VH having the sequence as shown in SEQ ID NO. 1 and the VL having the sequence as shown in SEQ ID NO. 11;
(i) the VH having the sequence as shown in SEQ ID NO. 1 and the VL having the sequence as shown in SEQ ID NO. 16;
(j) the VH having the sequence as shown in SEQ ID NO. 1 and the VL having the sequence as shown in SEQ ID NO. 19;
(k) the VH having the sequence as shown in SEQ ID NO. 6 and the VL having the sequence as shown in SEQ ID NO. 24; and
(l) the VH having the sequence as shown in SEQ ID NO. 6 and the VL having the sequence as shown in SEQ ID NO. 29.

In some embodiments, the monoclonal antibody further includes constant regions, wherein the constant regions are a kappa chain constant region Ckappa and a human IgG4 constant region CH1-CH3.

In some embodiments, the antibody or the antigen-binding fragment thereof is selected from a chimeric antibody, a humanized antibody, a monoclonal antibody, scFv, Fab, Fab', F(ab')2 and Fv fragments, disulfide-linked Fv (dsFv), a diabody, a bispecific antibody and a multispecific antibody.

In a second aspect, the present invention further provides a nucleic acid molecule, which is specifically a nucleic acid molecule encoding the antibody or the antigen-binding fragment thereof in the first aspect.

The nucleic acid molecule encoding the antibody heavy chain variable region is composed of a nucleotide sequence as shown in SEQ ID NO. 2 or 7, and the nucleic acid molecule encoding the antibody light chain variable region is composed of a nucleotide sequence as shown in SEQ ID NO. 23, 12, 17, 20, 25 or 30.

In a third aspect, the present invention further provides an expression vector, which specifically includes the nucleic acid molecule in the second aspect.

In a fourth aspect, the present invention further provides a host cell, which specifically includes the nucleic acid molecule in the second aspect or the expression vector in the third aspect.

The host cell is a HEK293 cell.

In a fifth aspect, the present invention further provides a preparation method for the antibody or the antigen-binding fragment thereof, which includes: culturing the host cell in the fourth aspect under conditions permitting expression of the antibody or the antigen-binding fragment thereof, and recovering the antibody or the antigen-binding fragment thereof from a culture of the cultured host cell.

In a sixth aspect, the present invention further provides a pharmaceutical composition, which includes the antibody or the antigen-binding fragment thereof in the first aspect, or the vector in the third aspect, or the host cell in the fourth aspect, and a pharmaceutically acceptable carrier and/or excipient.

In a seventh aspect, the present invention further provides a detection reagent or kit, which includes the antibody or the antigen-binding fragment thereof in the first aspect, or the vector in the third aspect, or the host cell in the fourth aspect, or the pharmaceutical composition in the sixth aspect, and an optional instruction for use.

In some embodiments, the detection reagent or kit includes the monoclonal antibodies h2D4H4K12 and/or h5H10H6K4.

A use of the antibody or the antigen-binding fragment thereof, the vector, the host cell, or the pharmaceutical composition above in manufacture of a drug for inhibition or reduction of activity of human CD132 also falls within the scope of protection of the present invention.

A use of the antibody or the antigen-binding fragment thereof, the vector, the host cell, or the pharmaceutical composition above in manufacture of a drug for prevention, neutralization or treatment of an autoimmune disease also falls within the scope of protection of the present invention.

A method for prevention, neutralization or treatment of an autoimmune disease by using the antibody or the antigen-binding fragment thereof, the vector, the host cell, or the pharmaceutical composition above also falls within the scope of protection of the present invention.

The autoimmune diseases are immune-related diseases associated with IL-4, IL-7, IL-9, IL-15 and/or IL-21 cytokine receptors, all of which are caused by aberrant activation of T cells, B cells or NK cells, which mistakenly attack autologous cells, including: systemic lupus erythematosus, rheumatoid arthritis, vitiligo, psoriasis, alopecia areata, inflammatory bowel disease, systemic sclerosis, aplastic anemia, and the like.

Further, a common subunit of the IL-4, IL-7, IL-9, IL-15 and/or IL-21 cytokine receptors is CD132. Therefore, the cytokine receptors above may be regulated by inhibiting or reducing the activity of the human CD132.

Specifically, in some embodiments of the present invention, in an in-vitro activity experiment for detecting an inhibitory activity of a humanized candidate antibody against the CD132, the humanized anti-human CD132 antibody h2D4H4K12 may inhibit an activity of IL-21-stimulated NK92 cells to release IFN-γ; and the humanized anti-human CD132 antibody h5H10H6K4 may inhibit an activity of IL-4-stimulated Ramos cells to express the CD23.

Further, the CD132 is also a key regulatory factor for functions of the T cells, B cells or NK cells. Therefore, the functions of the T cells, B cells or NK cells may be regulated by inhibiting or reducing the activity of the human CD132.

Specifically, in some embodiments, both the antibodies h2D4H4K12 and h5H10H6K4 exert inhibitory effects on the B cells, T cells and NK cells, but do not affect numbers of neutrophils, monocytes, erythrocytes and platelets.

Further, the antibodies h2D4H4K12 and/or h5H10H6K4 may be used for treatment of the autoimmune disease such as lupus erythematosus, vitiligo, psoriasis and graft-versus-host disease (GVHD).

Specifically, in some embodiments, both the antibodies h2D4H4K12 and h5H10H6K4 can significantly reduce urine protein and plasma anti-dsDNA levels, thereby improving a survival rate.

Specifically, in some embodiments, the antibody h2D4H4K12 can significantly reduce an area of vitiligo macules, thereby obviously improving a disease phenotype of vitiligo.

Specifically, in some embodiments, the antibody h2D4H4K12 can obviously alleviate disease symptoms of psoriasis.

Specifically, in some embodiments, the antibody h2D4H4K12 can reduce inflammatory cell infiltration in the liver, lung and kidney in the graft-versus-host disease.

Beneficial effects:
In the present invention, two antibodies (2D4 and 5H10) previously obtained in the laboratory are humanized, and then screened to obtain the two high-affinity humanized monoclonal antibodies (h2D4H4K12 and h5H10H6K4) capable of targeting the human CD132. Inhibitory activities of the two antibodies against 5 γc cytokines are consistent with those of the parental chimeric antibodies 2D4 and 5H10, and meanwhile, immunogenicities of the parental chimeric antibodies is reduced, thereby reducing the risk of potential immune responses to the antibodies in patients receiving the antibodies. Both the antibodies may be used in therapeutic drugs for prevention, neutralization or treatment of the autoimmune diseases related to the IL-4, IL-7, IL-9, IL-15 and/or IL-21 cytokines (such as systemic lupus erythematosus, rheumatoid arthritis, vitiligo, psoriasis, alopecia areata, inflammatory bowel disease, systemic sclerosis, graft-versus-host disease and aplastic anemia). Furthermore, the present invention demonstrates the significant inhibitory effects of the two antibodies on the T cells, B cells and NK cells in CD132 humanized mice, which indicates potential therapeutic effects of the antibodies against the autoimmune diseases caused by aberrant activation of the T cells, B cells and NK cells (such as systemic lupus erythematosus, rheumatoid arthritis, vitiligo, psoriasis, alopecia areata, inflammatory bowel disease, systemic sclerosis, graft-versus-host disease and aplastic anemia). The present invention further illustrates the promising prospects of applying the two antibodies in the treatment of the autoimmune diseases by using a systemic lupus erythematosus animal model, a vitiligo animal model, a psoriasis animal model and a graft-versus-host disease animal model.

### BRIEF DESCRIPTION OF THE DRAWINGS

The present invention is further described in detail hereinafter with reference to the drawings and specific embodiments, and the advantages of the above and/or other aspects of the present invention will be clearer.
FIG. 1 shows inhibition of IFN-γ secretion from NK92 cells by a parental chimeric anti-human CD132 antibody 2D4 and multiple humanized 2D4 antibodies under constant stimulation of IL-21.
FIG. 2 shows inhibition of CD23 expression on Ramos cells by a parental chimeric anti-human CD132 antibody 5H10 and multiple humanized 5H10 antibodies under constant stimulation of IL-4.
FIG. 3 shows inhibition of IL-21-stimulated IFN-γ secretion from the NK92 cells by h5H10H6K4.
FIG. 4 shows inhibition of IL-4-stimulated CD23 expression on the Ramos cells by 2D4H4K12.
FIG. 5 shows inhibition of IL15-stimulated IFN-γ secretion from the NK92 cells by the h5H10H6K4 (FIG. 5-1) and the h2D4H4K12 (FIG. 5-2).
FIG. 6 shows inhibition of IL-7-stimulated downstream STATS phosphorylation in CD4⁺ T cells by the h5H10H6K4 (FIG. 6-1) and the h2D4H4K12 (FIG. 6-2).
FIG. 7 shows inhibition of IL-9-stimulated proliferation of M07E cells by the h5H10H6K4 (FIG. 7-1) and the h2D4H4K12 (FIG. 7-2).
FIG. 8 shows immunogenicities of the h2D4H4K12 and the h5H10H6K4.
FIG. 9 shows effects of the h2D4H4K12 and h5H10H6K4 antibodies on T cells (9A), B cells (9B), NK cells (9C), neutrophils (9D), erythrocytes (9E), monocytes (9F), platelets (9G) and Treg cells (9H) in CD132^{hu/hu} background mice.
FIG. 10 shows effects of the h2D4H4K 12 and h5H10H6K4 antibodies on an anti-dsDNA level (10A) and a urine protein/creatinine ratio (10B) in a pristane-induced CD132^{hu/hu} lupus erythematosus mouse model.
FIG. 11 shows a therapeutic effect of the h2D4H4K12 on vitiligo.
FIG. 12 shows a therapeutic effect of the h2D4H4K12 on psoriasis.
FIG. 13 shows a therapeutic effect of the h2D4H4K12 on graft-versus-host disease (GVHD).

### DETAILED DESCRIPTION

All experimental methods described in the following embodiments are conventional methods unless otherwise specified; and all reagents and materials are commercially available unless otherwise specified.

In the previous studies of the research group, monoclonal chimeric antibodies 2D4 and 5H10 targeting human CD132 were obtained via phage library screening (detailed screening, construction and sequence information of the CD132-targeting chimeric antibodies 2D4 and 5H10 have been disclosed in patent CN116970081A). However, the chimeric antibodies are highly immunogenic and tend to induce the production of anti-antibodies in the body, thereby reducing therapeutic effects of the antibodies. Therefore, the present patent further describes humanization of the target antibodies 2D4 and 5H10 to reduce immunogenicities of the chimeric antibodies without attenuating antibody activities, and simultaneously performs in-vivo pharmacodynamic validation on the target antibodies (h2D4H4K12 and h5H10H6K4) to further the effectiveness of the two antibodies.

All experiments were approved and conducted in accordance with the guidelines of the Ethics Committee of the Hospital for Skin Diseases (Institute of Dermatology), Chinese Academy of Medical Sciences.

### Embodiment 1: Humanization of monoclonal antibodies 2D4 and 5H10

Variable region sequences of the monoclonal antibodies 2D4 and 5H10 were compared with sequences of human germline antibodies to identify highly homologous sequences for CDR grafting. Subsequently, homology modeling was performed with a computer to analyze CDR regions and surrounding framework amino acid sequences thereof, thereby investigating a spatial three-dimensional binding mode thereof. Key amino acid residues in gene sequences of positive monoclonal antibodies that may interact with CD132 and maintain a spatial framework were analyzed by calculating an electrostatic force, a van der Waals force, hydrophobicity and hydrophilicity, and an entropy, and back-mutation sites were designed on this basis. HLA-DR affinity was analyzed and investigated to select human germline framework sequences with low immunogenicity. The above humanization was designed and completed by Keymed Biosciences Co., Ltd.

A total of 1 heavy chain variable region derivative (h2D4-VH4) and 4 light chain variable region derivatives (h2D4-VL3, h2D4-VL9, h2D4-VL11 and h2D4-VL12) were designed for the antibody 2D4; and a total of 1 heavy chain variable region derivative (h5H10-VH6) and 2 light chain variable region derivatives (h5H10-VL3 and h5H10-VL4) were designed for the antibody 5H10. Sequences of all the above light and heavy chain variable region derivatives were shown in Table 1 and Table 2. The light chain variable region derivatives were cloned into a pHCT2 vector (purchased from Addgene) independently developed by Keymed Biosciences, which contained a human IgGκ light chain constant region and regulatory elements, so as to express full IgGκ light chains in mammalian cells. The heavy chain variable region derivatives were cloned into a pHCT1s vector (purchased from Addgene) independently developed by Keymed Biosciences, which contained a human IgG4 subtype heavy chain constant region and regulatory elements, so as to express full IgG4 subtype heavy chains in mammalian cells. Plasmids were paired and then transfected into HEK293 cells (purchased from ATCC), and expressed for 5-6 days, and then, a supernatant was harvested, filtered and purified by using a protein A column, so as to construct and obtain candidate humanized antibodies h2D4H4K3, h2D4H4K9, h2D4H4K11, h2D4H4K12, h5H10H6K4 and h5H10H6K5.

**Table 1 Sequences of heavy chain variable region derivatives of candidate CD132 humanized antibodies (SEQ ID NO.** omitted before numbers)**

| Heavy chain variable region derivative | HCVR | | HCDR1 | HCDR2 | HCDR3 |
|---|---|---|---|---|---|
| | Amino acid sequence | Nucleotide sequence | Amino acid Sequence | Amino acid Sequence | Amino acid Sequence |
| h2D4-VH4 | 1 | 2 | 3 | 4 | 5 |
| h5H10-VH6 | 6 | 7 | 8 | 9 | 10 |

**Table 2 Sequences of light chain variable region derivatives of candidate CD132 humanized antibodies (SEQ ID NO.** omitted before numbers)**

| Light chain variable region derivative | LCVR | | LCDR1 | LCDR2 | LCDR3 |
|---|---|---|---|---|---|
| | Amino acid sequence | Nucleotide sequence | Amino acid Sequence | Amino acid Sequence | Amino acid Sequence |
| h2D4-VL3 | 11 | 12 | 13 | 14 | 15 |
| h2D4-VL9 | 16 | 17 | 18 | 14 | 15 |
| h2D4-VL11 | 19 | 20 | 21 | 14 | 15 |
| h2D4-VL12 | 22 | 23 | 18 | 14 | 15 |
| h5H10-VL3 | 24 | 25 | 26 | 27 | 28 |
| h5H10-VL4 | 29 | 30 | 26 | 27 | 31 |

Specifically, the sequences of the heavy chain variable region derivatives of the humanized antibodies were as follows.
1) The amino acid sequence of the h2D4-VH4 was as shown in SEQ ID NO. 1, the encoding nucleotide sequence of the h2D4-VH4 was as shown in SEQ ID NO. 2, and CDR1, CDR2 and CDR3 of the h2D4-VH4 were as shown in SEQ ID NO. 3, SEQ ID NO. 4 and SEQ ID NO. 5 respectively.

<------------FR1------------> CDR1 <----FR2-----> CDR2
QVQLVQSGAEVKKPGSSVKVSCKTSGFNIEDIYLHWVRQAPGQGLEWMGRID
PANGK
<-------------FR3-------------> CDR3 <---FR4-

SNYDPKFQGRVTITADTSTSTAYMELSSLRSEDTAVYYCAALRFFGLDYWGQG
TLVT
-->

### VSS

### Nucleotide sequence

CAGGTTCAGCTGGTTCAGTCTGGCGCCGAAGTGAAGAAACCTGGCAGCAGC
GTGAAGGTGAGCTGCAAGACAAGCGGCTTCAACATCGAGGACATCTACCTGCAC
TGGGTCCGACAGGCTCCAGGACAGGGACTTGAGTGGATGGGCAGAATCGACCCT
GCCAACGGCAAGAGCAACTACGACCCCAAGTTCCAGGGCAGAGTCACCATCACA
GCCGACACCAGCACCAGCACCGCCTACATGGAACTGAGCAGCCTGAGAAGCGAG
GACACCGCCGTGTACTACTGTGCCGCTCTGAGATTCTTCGGCCTGGACTACTGGG
GCCAGGGAACACTGGTTACCGTGTCTAGT

2) The amino acid sequence of the h5H10-VH6 was as shown in SEQ ID NO. 6, the encoding nucleotide sequence of the h5H10-VH6 was as shown in SEQ ID NO. 7, and CDR1, CDR2 and CDR3 of the h5H10-VH6 were as shown in SEQ ID NO. 8, SEQ ID NO. 9 and SEQ ID NO. 10 respectively.

<-----------FR1-----------> CDR1 <----FR2-----> CDR2
QVQLVQSGAEVKKPGASVKVSCKASGYTFTNYWLGWVRQAPGQGLEWMGD
IYPGGGY

<------------FR3-------------> CDR3 <--FR4
TNYNEKFQGRVTMTADTSTSTVYMELSSLRSEDTAVYFCARGDYGSSWFPYW
GQGTL
---->

### VTVSS

### Nucleotide sequence

CAGGTTCAGCTGGTTCAGTCTGGCGCCGAAGTGAAGAAACCTGGCGCCTCC
GTGAAGGTGAGCTGCAAGGCTAGCGGCTACACCTTCACCAACTACTGGCTCGGC
TGGGTCCGACAGGCTCCTGGACAGGGACTGGAATGGATGGGCGACATCTACCCT
GGCGGCGGATACACAAACTACAACGAGAAGTTCCAGGGCAGAGTCACAATGACC

GCCGACACCTCTACCAGCACAGTCTACATGGAACTGAGCAGCCTGAGAAGCGAG
GATACCGCCGTGTACTTCTGTGCCAGAGGCGACTACGGCTCTAGCTGGTTTCCTTA
CTGGGGCCAGGGAACCCTGGTCACCGTTTCTTCT

Specifically, the sequences of the light chain variable region derivatives of the humanized antibodies were as follows.

3) The amino acid sequence of the h2D4-VL3 was as shown in SEQ ID NO. 11, the encoding nucleotide sequence of the h2D4-VL3 was as shown in SEQ ID NO. 12, and CDR1, CDR2 and CDR3 of the h2D4-VL3 were as shown in SEQ ID NO. 13, SEQ ID NO. 14 and SEQ ID NO. 15 respectively.

<----------FR1----------> CDR1 <-----FR2-----> CDR2 <-
DMQMTQSPSSLSASVGDRVTITCSATQSVSYMYWYQQKPGKAPELWIYRTSN
LASGV
-------------FR3--------------> CDR3 <--FR4--->
PSRFSGSGSGTDYTFTISSLQPEDIATYYCQQWSSNPPTFGQGTKLEIK

### Nucleotide sequence

GACATGCAGATGACACAGAGCCCTTCTAGCCTGTCTGCCAGCGTGGGCGAC
AGAGTGACCATCACATGTAGCGCCACACAGAGCGTGTCCTACATGTACTGGTATC
AGCAGAAGCCCGGCAAGGCCCCTGAGCTTTGGATCTACAGAACAAGCAACCTGG
CCAGCGGCGTGCCCTCTAGATTTCTGGCTCTGGCAGCGGCACCGACTACACCTT
CACAATCTCTAGCCTGCAGCCTGAGGATATCGCCACCTACTACTGCCAGCAGTGG
TCTAGCAACCCTCCTACATTCGGCCAGGGCACCAAGCTGGAAATCAAG

4) The amino acid sequence of the h2D4-VL9 was as shown in SEQ ID NO. 16, the encoding nucleotide sequence of the h2D4-VL9 was as shown in SEQ ID NO. 17, and CDR1, CDR2 and CDR3 of the h2D4-VL9 were as shown in SEQ ID NO. 18, SEQ ID NO. 14 and SEQ ID NO. 15 respectively.

<---------FR1---------> CDR1 <-----FR2-----> CDR2 <-
EIVLTQSPATLSLSPGERATLSCRATSSVSYMYWYQQKPGQAPRLLIYRTSNLAS
GI
-------------FR3-------------> CDR3 <--FR4--->

PARFSGSGSGTDYTLTISSLEPEDFAVYYCQQWSSNPPTFGQGTKLEIK

### Nucleotide sequence

GAGATCGTGCTGACACAGAGCCCTGCCACACTGTCACTGTCTCCAGGCGAG
AGAGCCACACTGAGCTGTCGCGCCACCAGCAGCGTGTCCTACATGTACTGGTATC
AGCAGAAGCCCGGCCAGGCTCCTCGGCTTCTGATCTACAGAACAAGCAACCTGG
CCAGCGGCATCCCCGCTAGATTTCTGGCTCTGGCAGCGGCACCGACTACACCCT
GACAATCTCTAGCCTGGAACCTGAGGACTTCGCCGTGTACTACTGCCAGCAGTGG
TCTAGCAACCCTCCTACATTCGGCCAGGGCACCAAGCTGGAAATCAAG

5) The amino acid sequence of the h2D4-VL11 was as shown in SEQ ID NO. 19, the encoding nucleotide sequence of the h2D4-VL11 was as shown in SEQ ID NO. 20, and CDR1, CDR2 and CDR3 of the h2D4-VL11 were as shown in SEQ ID NO. 21, SEQ ID NO. 14 and SEQ ID NO. 15 respectively.

<---------FR1---------> CDR1 <-----FR2-----> CDR2 <-
DIQMTQSPSSLSASVGDRVTITCQATQSVSYMYWYQQKPGKAPELLIYRTSNL
ASGV
-------------FR3-------------> CDR3 <--FR4--->
PSRFSGSGSGTDYTFTISSLQPEDIATYYCQQWSSNPPTFGQGTKLEIK

### Nucleotide sequence

GACATCCAGATGACACAGAGCCCTTCTAGCCTGTCTGCCAGCGTGGGCGACA
GAGTGACCATCACATGTCAGGCCACACAGAGCGTGTCCTACATGTACTGGTATCA
GCAGAAGCCCGGCAAGGCCCCTGAGCTTCTGATCTACAGAACAAGCAACCTGGC
CAGCGGCGTGCCCTCTAGATTTTCTGGCTCTGGCAGCGGCACCGACTACACCTTC
ACAATCTCTAGCCTGCAGCCTGAGGATATCGCCACCTACTACTGCCAGCAGTGGT
CTAGCAACCCTCCTACATTCGGCCAGGGCACCAAGCTGGAAATCAAG

6) The amino acid sequence of the h2D4-VL12 was as shown in SEQ ID NO. 22, the encoding nucleotide sequence of the h2D4-VL12 was as shown in SEQ ID NO. 23, and CDR1, CDR2 and CDR3 of the h2D4-VL12 were as shown in SEQ ID NO. 18, SEQ ID NO. 14 and SEQ ID NO. 15 respectively.

<-----------FR1----------->     CDR1 <------FR2------> CDR2 <-

EIVLTQSPATLSLSPGERATLSCRATSSVSYMYWYQQKPGQAPELLIYRTSNLAS
GI
-------------FR3------------->    CDR3 <--FR4--->
PARFSGSGSGTDYTLTISSLEPEDFAVYYCQQWSSNPPTFGQGTKLEIK

### Nucleotide sequence

GAGATCGTGCTGACACAGAGCCCTGCCACACTGTCACTGTCTCCAGGCGAG
AGAGCCACACTGAGCTGTCGCGCCACCAGCAGCGTGTCCTACATGTACTGGTATC
AGCAGAAGCCCGGCCAGGCTCCTGAGCTTCTGATCTACAGAACAAGCAACCTGG
CCAGCGGCATCCCCGCTAGATTTCTGGCTCTGGCAGCGGCACCGACTACACCCT
GACAATCTCTAGCCTGGAACCTGAGGACTTCGCCGTGTACTACTGCCAGCAGTGG
TCTAGCAACCCTCCTACATTCGGCCAGGGCACCAAGCTGGAAATCAAG

7) The amino acid sequence of the h5H10-VL3 was as shown in SEQ ID NO. 24, the encoding nucleotide sequence of the h5H10-VL3 was as shown in SEQ ID NO. 25, and CDR1, CDR2 and CDR3 of the h5H10-VL3 were as shown in SEQ ID NO. 26, SEQ ID NO. 27 and SEQ ID NO. 28 respectively.

<---------FR1---------> CDR1 <-----FR2-----> CDR2 <-
DIQMTQSPSSLSASVGDRVTITCRASQSISNNLHWYQQKPGKAPKLLIKYASQSI
SG
-------------FR3-------------> CDR3 <--FR4--->
VPSRFSGSGSGTDFTLTISSLQPEDFATYFCQQSDSWLTFGQGTKVEIK

### Nucleotide sequence

GACATCCAGATGACACAGAGCCCTTCTAGCCTGTCTGCCAGCGTGGGCGACA
GAGTGACCATCACATGTAGAGCCAGCCAGAGCATCAGCAACAACCTGCACTGGT
ATCAGCAGAAGCCCGGCAAGGCTCCCAAGCTGCTGATTAAGTACGCCAGCCAGT
CCATCTCCGGCGTGCCATCTAGATTCAGCGGCTCTGGCTCTGGCACCGACTTCAC
CCTGACAATCTCTAGCCTGCAGCCTGAGGACTTCGCTACCTACTTCTGCCAGCAG
AGCGACAGCTGGCTGACATTTGGCCAGGGCACCAAGGTGGAAATCAAG

8) The amino acid sequence of the h5H10-VL4 was as shown in SEQ ID NO. 29, the encoding nucleotide sequence of the h5H10-VL4 was as shown in SEQ ID NO. 30, and CDR1, CDR2 and CDR3 of the h5H10-VL4 were as shown in SEQ ID NO. 26, SEQ ID NO. 27 and SEQ ID NO. 31 respectively.

<----------FR1---------> CDR1 <-----FR2-----> CDR2 <-
DIQMTQSPSSLSASVGDRVTITCRASQSISNNLHWYQQKPGKAPKLLIKYASQSI
SG
--------------FR3-------------> CDR3 <--FR4--->
VPSRFSGSGSGTDFTLTISSLQPEDFATYFCQQSDSYLTFGQGTKVEIK

### Nucleotide sequence

GACATCCAGATGACACAGAGCCCTTCTAGCCTGTCTGCCAGCGTGGGCGACA
GAGTGACCATCACATGTAGAGCCAGCCAGAGCATCAGCAACAACCTGCACTGGT
ATCAGCAGAAGCCCGGCAAGGCTCCCAAGCTGCTGATTAAGTACGCCAGCCAGT
CCATCTCCGGCGTGCCATCTAGATTCAGCGGCTCTGGCTCTGGCACCGACTTCAC
CCTGACAATCTCTAGCCTGCAGCCTGAGGACTTCGCTACCTACTTCTGCCAGCAG
AGCGACAGCTACCTGACATTTGGCCAGGGCACCAAGGTGGAAATCAAG

### Embodiment 2: In-vitro activity experiment for inhibitory activities of candidate humanized antibodies against CD132

In order to further evaluate a difference in activity between a candidate humanized antibody and a parental chimeric antibody, an experimental method described in the patent CN116970081A was referenced. Since a parental chimeric antibody 2D4 had strong inhibitory activity against IL-21, a 2D4 candidate humanized antibody was evaluated by a method of IL-21-stimulated IFN-γ secretion from a NK92 cell line. Since a difference in activity between 5H10 candidate humanized antibodies was more significant in an inhibition experiment system of IL-4, the 5H10 candidate humanized antibody was further evaluated by a method of IL-4-stimulated CD23 up-regulation on a Ramos cell line.

2.1 Validation on inhibition of IFN-γ secretion from NK92 cells by 2D4 humanized anti-human CD132 candidate antibody

The NK-92 cells (Procell CL-0530) were seeded in a 96-well plate at 2×10⁴ cells per well by using a growth medium (prepared according to Procell's instruction, without IL-2), and incubated in 5% CO₂ at 37°C overnight for starvation. On the next day, each 2D4 humanized anti-human CD132 candidate antibody was serially diluted 3-fold from 400 nM to 1.6 nM in the growth medium (without IL-2) respectively, and added to the NK-92 cells and incubated for 30 minutes. After incubation, 250 pM IL-21 was added to the NK-92 cells containing different 2D4 humanized anti-human CD132 candidate antibodies respectively. After incubation in 5% CO₂ at 37°C for 72 hours, IFN-γ secretion was determined by ELISA.

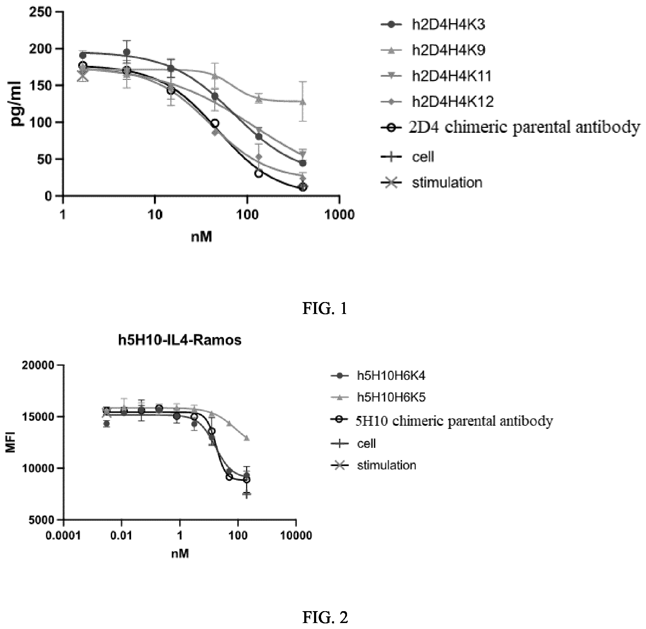

A human IFN-γ ELISA kit (BD Cat. No. 555142) was used, and a capture antibody was coated onto a 96-well microplate at 1: 250 and incubated at 4°C overnight. On the next day, the plate was washed 3 times with a PBS buffer (pH 7.2), blocked with 10% FBS at room temperature for 1 hour, and washed another 3 times with a PBS buffer (pH 7.2). Subsequently, 100 µL of samples (cell supernatants) and standards were added respectively, and incubated at room temperature for 2 hours. Subsequently, the plate was washed 5 times with a PBS buffer (pH 7.2), and 5AV-HRP was added to a dilution containing 0.4% detection antibody at 1: 250. Finally, after incubation at room temperature for 1 hour, the plate was washed 7 times with a PBS buffer (pH 7.2), and 100 µL of chromogenic solution (TMB solution, Sigma Cat. No. T2885) was added. After placement at 37°C for 10 minutes, 50 µL of 2 M concentrated sulfuric acid solution was added to terminate the reaction, and the reaction product was immediately placed in a microplate reader to obtain an OD₄₅₀ value. FIG. 1 indicated that the inhibitory activity of the humanized anti-human CD132 antibody h2D4H4K12 against IL-21-stimulated IFN-γ release from the NK92 cells was comparable to that of the parental 2D4 chimeric antibody.

2.2 Validation on inhibition of IL-4-stimulated CD23 expression on Ramos cells by 5H10 humanized anti-CD132 candidate antibody

The IL-4 could induce CD23 expression in the Ramos cells, so that this characteristic could be used to evaluate the inhibitory effect of the 5H10 humanized anti-CD132 candidate antibody on IL-4 signal transduction. The Ramos cell line (ATCC) was cultured in a RPMI 1640 complete medium containing 10% FBS in 5% CO₂ at 37°C, with a cell concentration controlled at 2×10⁵-2×10⁶ cells per 1 ml, and seeded into a 96-well flat-bottom cell culture plate at 100 µL per well. Serial dilutions of each 5H10 humanized anti-CD132 candidate antibody were prepared in the RPMI 1640 complete medium, with 4-fold serial dilutions from 200 nM to 0.003 nM. After co-incubation with the cells for 30 minutes, 50 µL of IL-4 at a final concentration of 2.67 nM was added, and cultured in 5% CO₂ at 37°C for 48 hours.

The above cultured cells were transferred to a 96-well U-bottom cell culture plate, and centrifuged at 300 g and 4°C for 3 minutes, and the supernatant was discarded. The cells were washed twice with a flow cytometry buffer (PBS buffer containing 4% calf serum, pH 7.2) at 200 µL per well. 50 µL of blocking solution (buffer containing 100 µg/mL hIgG) was added to the cells and blocked in an ice bath for 10 minutes, and the supernatant was discarded after centrifugation at 300 g for 3 minutes. Subsequently, an anti-human CD23 FITC antibody dilution (BD EBVCS-5) was added at 50 µL per well and blocked in an ice bath for 20 minutes, and the supernatant was discarded after centrifugation at 300 g for 3 minutes. Subsequently, the cells were washed twice with a flow cytometry buffer at 200 µL per well. A PI staining solution was added to the cells at 100 µL per well and incubated in an ice bath in the dark for 5 minutes, and the supernatant was discarded after centrifugation at 300 g for 3 minutes. Subsequently, the cells were washed twice with a flow cytometry buffer at 200 µL per well, and then resuspended with a PBS buffer (pH 7.2) at 100 µL per well. A mean fluorescence intensity was measured with a flow cytometer, and results were recorded. The results were shown in FIG. 2 and indicated that the inhibitory activity of the humanized anti-human CD132 antibody h5H10H6K4 against IL-4-stimulated CD23 expression on the Ramos cells was comparable to that of the parental 5H10 chimeric antibody.

### Embodiment 3: Re-validation of humanized anti-human CD132 antibodies h5H10H6K4 and h2D4H4K12

In Embodiment 2, the inhibitory activities of the candidate humanized antibodies against CD132 were determined by the in-vitro activity experiment, and two humanized anti-human CD132 antibodies h5H10H6K4 and h2D4H4K12 were screened and obtained. In this embodiment, the inhibitory activities of the two antibodies against 5 γc cytokines were further re-validated to determine whether the antibodies were consistent with those of the parental chimeric antibodies 2D4 and 5H10.

3.1 Inhibition of IL-21-stimulated IFN-γ secretion from NK92 cells by humanized anti-human CD132 antibody h5H10H6K4

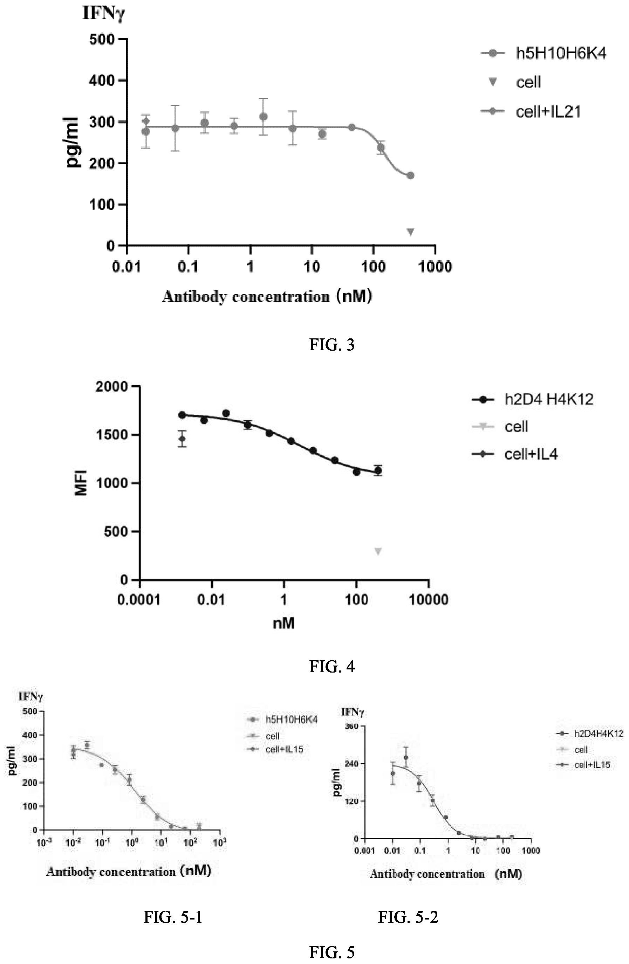

In order to further evaluate an ability of the h5H10H6K4 to block IL-21-stimulated IFN-y secretion from the NK92 cells, specific experimental steps referred to Embodiment 2.1, except that the antibody was replaced with the h5H10H6K4. Results were shown in FIG. 3, and FIG. 3 indicated that the anti-human CD132 antibody h5H10H6K4 could inhibit IL-21-stimulated IFN-γ release from the NK92 cells. However, compared with the results of the h2D4H4K12 in FIG. 1, the inhibitory activity of this antibody was weaker than that of the h2D4H4K12.

3.2 Inhibition of IL-4-stimulated CD23 expression on Ramos cells by humanized anti-human CD132 antibody h2D4H4K12

In order to further evaluate an ability of the h2D4H4K12 to block IL-4-stimulated CD23 expression on the Ramos cells, specific experimental steps referred to Embodiment 2.2, except that the antibody was replaced with the h2D4H4K12. The results were shown in FIG. 4, and FIG. 4 indicated that the anti-human CD132 antibody h2D4H4K12 could inhibit IL-4-stimulated CD23 expression on the Ramos cells.

3.3 Inhibition of IL15-stimulated IFN-γ secretion from NK92 cells by humanized anti-human CD132 antibodies h2D4H4K12 and h5H10H6K4

In order to further evaluate abilities of the h2D4H4K12 and h5H10H6K4 to block IL-15-stimulated IFN-γ secretion from the NK92 cells, specific experimental steps referred to Embodiment 2.1, wherein the antibodies h5H10H6K4 and h2D4H4K12 were used respectively, and 250 pM IL-21 was replaced with 2.5 nM IL-15. Experimental results were shown in FIG. 5-1 and FIG. 5-2. FIG. 5-1 and FIG. 5-2 respectively indicated that both the humanized anti-human CD132 antibodies h5H10H6K4 and h2D4H4K12 could inhibit IL-15-stimulated IFN-γ release from the NK92 cells. However, the inhibitory activity of the h5H10H6K4 was weaker than that of the h2D4H4K12.

3.4 Flow cytometric analysis of STAT phosphorylation in human CD4⁺ T cells (human PBMC)

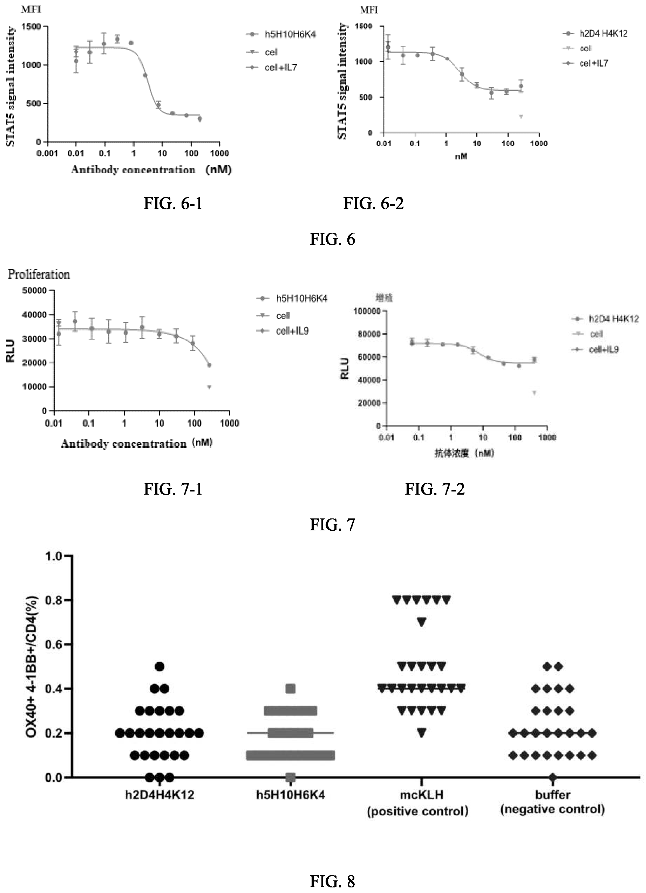

In order to further evaluate in-vitro properties of the humanized anti-human CD132 antibodies h5H10H6K4 and h2D4H4K12, abilities of the antibodies to block IL-7-induced activation of the CD4⁺ T cell were further determined by flow cytometry of protein phosphorylation. The experimental procedure was the same as that in Embodiment 8 of the patent CN116970081A, except that the chimeric anti-human CD132 antibodies 5H10 and 2D4 were replaced with the humanized anti-human CD132 antibodies h5H10H6K4 and h2D4H4K12. Experimental results were shown in FIG. 6-1 and FIG. 6-2. FIG. 6-1 and FIG. 6-2 respectively indicated that the anti-human CD132 antibodies h5H10H6K4 and h2D4H4K12 could inhibit IL-7-stimulated downstream STATS phosphorylation in human peripheral blood CD4⁺ T cells.

3.5 Inhibition of IL-9-stimulated proliferation of M07E cells by humanized anti-human CD132 antibodies h2D4H4K12 and h5H10H6K4

The inhibitory activities of the humanized anti-human CD132 antibodies h5H10H6K4 and h2D4H4K12 against the IL-9 were further evaluated. The experimental procedure was the same as that in Embodiment 10 of the patent CN116970081A, except that the chimeric anti-human CD132 antibodies 5H10 and 2D4 were replaced with the humanized anti-human CD132 antibodies h5H10H6K4 and h2D4H4K12. Results were shown in FIG. 7, and FIG. 7-1 and FIG. 7-2 respectively indicated that the humanized anti-human CD132 antibodies h5H10H6K4 and h2D4H4K12 could inhibit IL-9-stimulated proliferation of the M07E cells.

### Embodiment 4: Evaluation of immunogenicities of humanized anti-human CD132 antibodies h2D4H4K12 and h5H10H6K4

Sample preparation:
Under sterile conditions, the h2D4H4K12 and h5H10H6K4, and KLH (independently expressed by Keymed Biosciences) were diluted to 200 µg/mL with an X-vivo 15 medium (Shanghai BasalMedia), and sterilized for later use.

Determination method:
Peripheral venous blood was collected from 25 healthy volunteers, and PBMCs were harvested by Ficoll (Healthcare) centrifugal separation. After washing once with PBS, the cells were counted, a cell density was adjusted to 2.5×10⁵/mL with the X-vivo 15 medium, and 100 µL was added to a 96-well U-bottom plate (corning). 100 µL of diluted sample was added to the cells, and incubated in 5% CO₂ at 37°C for 48 hours, KLH was used as a positive control, and a well without the sample was used as a negative control. The groups were determined by the same method. The supernatant was discarded from the 96-well plate at 200 µL/well, then the cells were washed once with a flow cytometry buffer (prepared from PBS+10%FBS), and 100 µg/mL hIgG (Jackson) was added at 50 µL per well, and incubated on ice for 30 minutes. FITC anti-human CD4, APC anti-human CD137 (4-1BB) and PE anti-human CD134 (OX40) fluorescent antibodies (all from BioLegend) were added at 1 µL/well, and incubated on ice for 45 minutes. 50 µL of 5 µg/mL PI was added to stain the cells for 5 minutes, and the cells were washed twice with a flow cytometry buffer, resuspended in 100 µL of PBS, and analyzed with a flow cytometer.

Data analysis:
(1) Lymphocytes were gated based on a cell size (FSC-A) and a granularity (SSC-A).
(2) Cell doublets were excluded from a lymphocyte population based on FSC-A and FSC-H, and a single-cell population was gated.
(3) A live-cell population was gated from the single-cell population based on FSC-A and a PI signal.
(4) CD4-positive T cells were gated from live cells, and a proportion of CD137 (4-1BB) and CD134 (OX40) double-positive cells in the cell population was analyzed.

Final results were shown in FIG. 8, and indicated that the double-positive cells of the humanized anti-human CD132 antibodies h2D4H4K12 and h5H10H6K4 were comparable to those of the negative control, thereby indicating that the two antibodies had extremely low immunogenicities.

### Embodiment 5: In-vivo immunosuppression experiment for evaluating effects of anti-human CD132 antibodies h2D4H4K12 and h5H10H6K4 on immune cell population in blood

Experimental procedure: C57BL/6 background mice (genetically modified C57BL/6 background mice from the breeding colony of Biocytogen, in which a full-length domain of endogenous CD132 was replaced with a corresponding human sequence) were subcutaneously administered with the antibodies h2D4H4K12 or h5H10H6K4 at a dose of 8 mg/kg or 16 mg/kg once every 4 days for 3 consecutive administrations, and mice without administration of the h2D4H4K12 or h5H10H6K4 were used as a control group.

**Table 3 Experimental drug administration and treatment protocol for mice in each group**

| Group | Strain of recipient | Number of mice per group | Antibody treatment |
|---|---|---|---|
| Group A | CD132^{hu/hu} | 6 | h2D4H4K12, 8 mg/kg |
| Group B | CD132^{hu/hu} | 6 | h2D4H4K12, 16 mg/kg |
| Group C | CD132^{h/hu} | 6 | h5H10H6K4, 8 mg/kg |
| Group D | CD 132^{hu/hu} | 6 | h5H10H6K4, 16 mg/kg |
| Group E | CD132^{hu/hu} | 6 | No administration |

The immune cell population in blood was analyzed within a period of time by flow cytometry. A specific procedure was as follows: numbers of total immune cells, B cells, T cells, NK cells and neutrophils in peripheral blood were analyzed by flow cytometry at different time points (once every 10 days) to evaluate effects of the antibodies h2D4H4K12 and h5H10H6K4 on absolute numbers of these types of cells. Briefly, at each time point, blood samples were collected from each mouse, and 50-100 µL of each blood sample was incubated in a red blood cell lysis buffer (Beyotime) at room temperature for 10 minutes to lyse red blood cells. If the lysis was insufficient, a second round of lysis was performed. After washing twice in a PBS buffer (Shanghai BasalMedia, pH 7.2), mouse IgG (Jackson Immunoresearch) was diluted to 200 µg/mL with a flow buffer (4% FBS (ExCell Bio)) to prepare an Fc receptor blocking solution. 50 µL of blocking solution was added to each lysed blood sample, and blocked at room temperature for 10 minutes. Subsequently, the cells were stained for cell surface markers by adding a diluted and fluorescently labeled antibody mixture (as described in Table 4) to a flow buffer, so as to identify CD45⁺ cells, T cells, B cells and NK cells (absolute numbers of neutrophils, monocytes, red blood cells and platelets were determined with a mouse hematology analyzer). Finally, the samples were washed twice in a flow buffer and resuspended in a PBS buffer (pH 7.2), and data of the samples were acquired with a BD celesta flow cytometer. Data analysis was performed with FlowJo v10 software. The CD45⁺ immune cells were restrained to singlet and live cells. Within this population, the T cells were further restrained to CD3⁺, the Treg cells were further restrained to CD4⁺ and CD25⁺, the B cells were further restrained to CD3⁻ and CD19⁺, and the NK cells were further restrained to CD3⁻, CD19⁻and NK1.1⁺.

**Table 4 Antibodies used in flow cytometry analysis**

| Antibody | Fluorescent antibody | Manufacturer | Final dilution degree |
|---|---|---|---|
| CD45 | PE | BD | 1: 400 |
| CD3 | APC-Cy7 | BD | 1: 200 |
| CD4 | FITC | Biolegend | 1: 200 |
| CD8a | BV510 | BD | 1: 200 |
| CD19 | APC | Biolegend | 1: 200 |
| CD25 | PerCP-Cy5.5 | BD | 1: 200 |
| NK1.1 | BV650 | BD | 1: 200 |

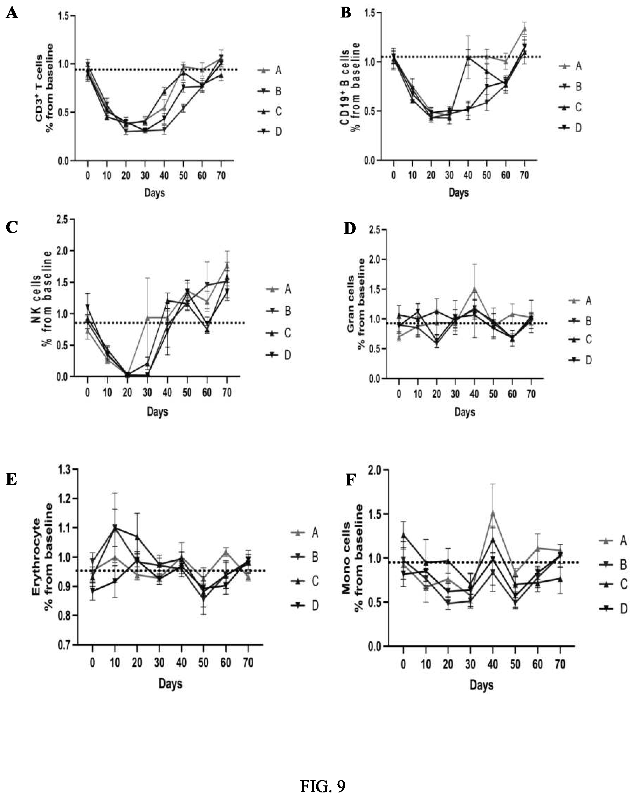

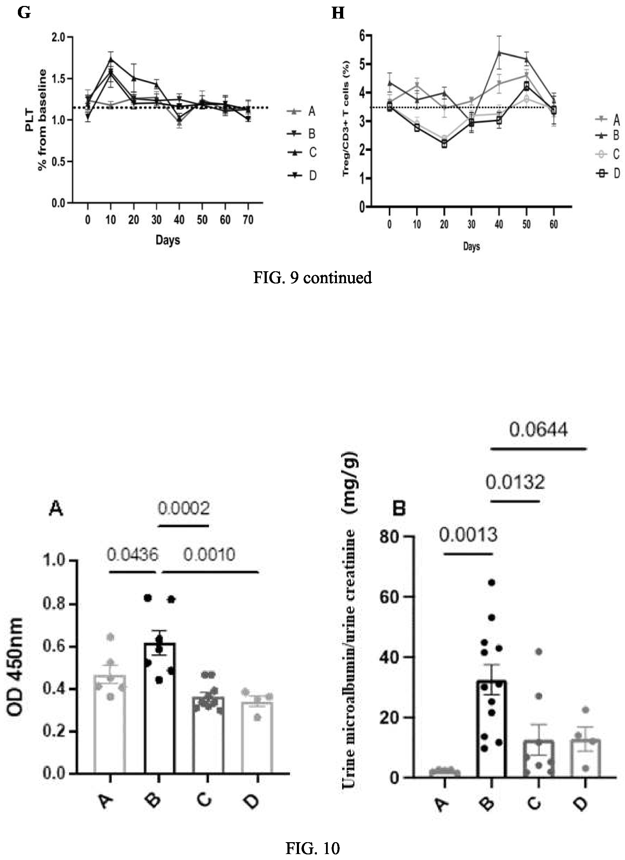

Results were shown in FIG. 9: the background mice were treated with the h2D4H4K12 or h5H10H6K4 (at 8 mg/kg and 16 mg/kg), resulting in significantly decreased numbers of the T cells (FIG. 9A), the B cells (FIG. 9B) and the NK cells (FIG. 9C) in blood of the mice, while the numbers of the neutrophils (FIG. 9D), the red blood cells (FIG. 9E), the monocytes (FIG. 9F) and the platelets (FIG. 9G) were unaffected, wherein the h2D4H4K12 had no obvious effect on the proportion of the Treg cells (FIG. 9H). After completion of 3 administrations, serum concentrations of the h2D4H4K12 and h5H10H6K4 were reduced over time. By the end of the study, all these data had returned to similar levels observed before treatment. The results indicated that both the h2D4H4K12 and h5H10H6K4 exerted inhibitory effects on the B cells, the T cells and the NK cells to varying degrees, but did not affect the numbers of the neutrophils, the monocytes, the red blood cells and the platelets. Furthermore, the inhibitory effect of the h2D4H4K12 on the Treg cells was weaker than that of the h5H10H6K4.

### Embodiment 6: Evaluation of therapeutic effects of anti-human CD132 antibodies h2D4H4K12 and h5H10H6K4 in pristane-induced lupus erythematosus mouse model

Experimental procedure: C57BL/6 background mice (genetically modified C57BL/6 background mice from the breeding colony of Biocytogen, in which a full-length domain of endogenous CD132 was replaced with a corresponding human sequence) were intraperitoneally injected with 0.5 mL of pristane (MCE), and mice in the control group were injected with an equal volume of PBS buffer (pH 7.2). 3 days before modeling, the mice were subcutaneously administered with the h2D4H4K12 or h5H10H6K4, or an anti-KLH isotype control (all the above antibodies were produced by Keymed Biosciences Co., Ltd.) at a dose of 20 mg/kg twice per week for 6 consecutive weeks, or the mice were not administered with the antibodies (Table 5). Urine protein, urine creatinine and plasma anti-dsDNA levels in the mice were monitored. The mice were sacrificed by cervical dislocation at week 12.

Specific monitoring experimental steps:
1. Urine microalbumin of the mice were detected with a mouse urine microalbumin ELISA kit (Elabscience): 20 µL of urine was collected from each mouse and detected according to operating steps. Briefly, the urine of each mouse was diluted 1000-fold with a standard/sample diluent, and 100 µL was added to a pre-coated 96-well ELISA plate in the kit, and incubated at 37°C for 90 minutes. After liquid removal, 100 µL of biotinylated antibody working solution was added to each well, and incubated at 37°C for 1 hour. The plate was washed 4 times, and 100 µL of HRP-conjugate working solution was added to each well, and incubated at 37°C for 30 minutes. The plate was washed 4 times, and 90 µL of chromogenic solution was added, and placed at 37°C for 10 minutes. Subsequently, 50 µL of stop solution was added, and the mixture was immediately placed in a microplate reader to obtain an OD₄₅₀ value.
2. Anti-dsDNA levels in plasma of the mice were detected by ELISA: 50-100 µL of blood was collected from each mouse and centrifuged at 4500 rpm, and the supernatant was frozen and preserved at -80°C to facilitate subsequent detection. A 96-well ELISA plate was coated with UltraPure^{™} salmon sperm DNA solution (Invitrogen) at 100 µg/mL, and incubated at 4°C overnight. On the next day, the plate was washed 3 times with a PBS buffer (pH 7.2), then blocked with 10% BSA (Sangon Biotech) at 37°C for 1 hour, and washed another 3 times with a PBS buffer (pH 7.2). Subsequently, plasma samples diluted 50-fold were added at 100 µL per well respectively, and incubated at 37°C for 90 minutes. Subsequently, the plate was washed 5 times with a PBS buffer (pH 7.2), and HRP-labeled Fc-specific anti-mouse IgG1 (abclonal) was added at a ratio of 1: 4000. After incubation at room temperature for 1 hour, the plate was washed 7 times with a PBS buffer (pH 7.2), and 100 µL of chromogenic solution (TMB solution, Sigma Cat. No. T2885) was added. After placement at 37°C for 10 minutes, 50 µL of 2 M concentrated sulfuric acid solution was added to terminate the reaction, and the reaction product was immediately placed in a microplate reader to obtain an OD₄₅₀ value.

**Table 5 Experimental drug administration and treatment protocol for mice in each group**

| Group | Strain of recipient | Number of mice per group | Modeling | mAb treatment |
|---|---|---|---|---|
| Group A | CD132^{hu/hu} | 5 | Injection with PBS | No mAb |
| Group B | CD132^{hu/hu} | 10 | Injection with pristane | anti KLH (isotype) |
| Group C | CD132^{hu/hu} | 10 | Injection with pristane | h2D4H4K 12 |
| Group D | CD132^{hu/hu} | 10 | Injection with pristane | h5H10H6K4 |

Results were shown in FIG.10: the treatment with the h2D4H4H4K12 or h5H10H6K4 antibody could reduce a content of auto-antibody anti-dsDNA in plasma of the mice (FIG. 10A) and reduce a urine protein/creatinine ratio of the mice (FIG. 10B). The results indicated that both the h2D4H4K12 and h5H10H6K4 antibodies could significantly reduce urine protein and plasma anti-dsDNA levels of pristane-induced lupus mice, so as to improve a survival rate of the mice, thereby indicating that both the h2D4H4K12 and h5H10H6K4 antibodies could effectively treat lupus erythematosus.

### Embodiment 7: Evaluation of therapeutic effect of anti-human CD132 antibody h2D4H4K12 in vitiligo mouse model

Experimental procedure: on day 0, C57BL/6 background mice (genetically modified C57BL/6 background mice from the breeding colony of Biocytogen, in which a full-length domain of endogenous CD132 was replaced with a corresponding human sequence) were intradermally injected with 2×10⁵ B16F10 cells (melanoma cells, purchased from Wuhan Procell Life Science and Technology Co., Ltd.). On day 4 and day 10, the mice were subcutaneously administered with anti-CD4 (purchased from Bio X cell) at a dose of 10 mg/kg twice respectively to deplete Treg cells in the mice. On day 12, melanomas were surgically removed. On day 30, the disease onset of the mice was observed, and mice with successful vitiligo modeling were selected and divided into two groups. A group A was subjected to subcutaneous administration with PBS to serve as an untreated control group, and a group B was subjected to subcutaneous administration with the h2D4H4K12 at the dose of 10 mg/kg twice per week for 4 consecutive weeks (Table 6). Areas of vitiligo macules in the mice were photographed and recorded before and after treatment.

**Table 6 Experimental drug administration and treatment protocol for mice in each group**

| Group | Strain of recipient | Number of mice per group | Modeling | mAb treatment |
|---|---|---|---|---|
| Group A | CD132^{hu/hu} | 3 | Subcutaneous injection with B16F10 | No mAb (PBS) |
| Group B | CD132^{hu/hu} | 3 | Subcutaneous injection with B16F10 | h2D4H4K 12 |

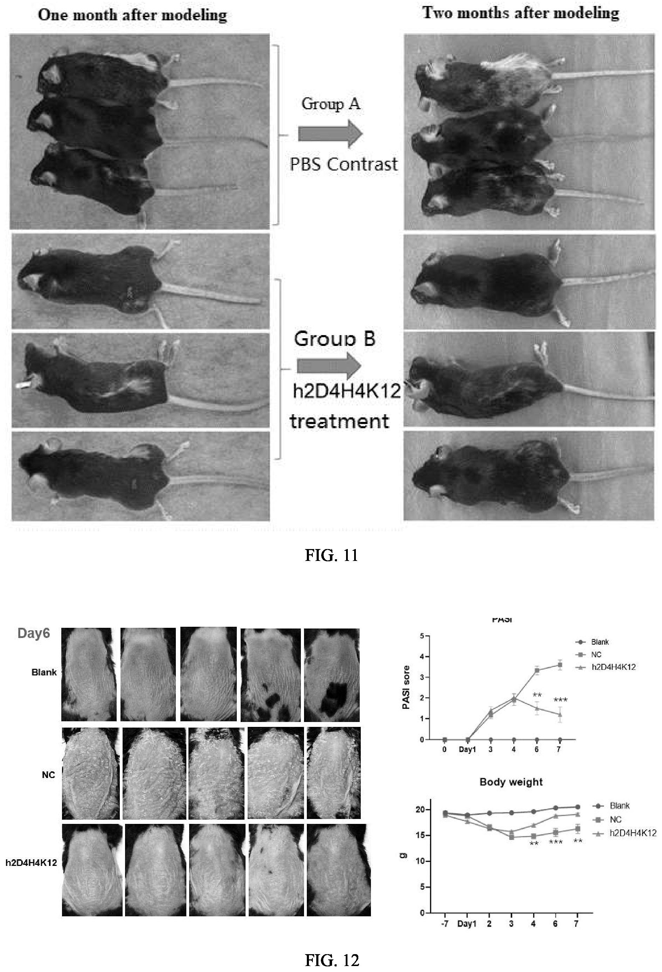

Results were shown in FIG. 11: after the disease onset of the vitiligo model mice, the mice in the PBS treatment group (group A) following one month of treatment exhibited a progressed state of vitiligo, and a significantly increased area of vitiligo macules, while the mice in the h2D4H4K12 treatment group (group B) exhibited a significantly reduced area of vitiligo macules. The results indicated that the h2D4H4K12 could effectively alleviate the symptoms of vitiligo.

### Embodiment 8: Evaluation of therapeutic effect of anti-human CD132 antibody h2D4H4K12 in psoriasis mouse model

Experimental procedure: on day 0, C57BL/6 background mice (genetically modified C57BL/6 background mice from the breeding colony of Biocytogen, in which a full-length domain of endogenous CD132 was replaced with a corresponding human sequence) were subjected to dorsal hair removal (2×3 cm), and subcutaneously administered with 300 µg of h2D4H4K12. From day 1 to day 5, 5% imiquimod cream was continuously applied to shaved dorsal skin once per day at a dose of 62.5 mg per application, body weights of the mice were measured, and dorsal skin lesions and rash inflammation were observed. On day 7, PASI scoring was performed, the mice were sacrificed, and the dorsal skin of the mice was collected and subjected to HE staining. Subsequently, abnormal keratinization, inflammatory infiltration, and the like were observed. The experimental mice were grouped as shown in Table 7 below.

**Table 7 Experimental drug administration and treatment protocol for mice in each group**

| Group | Strain of recipient | Number of mice per group | Modeling | mAb treatment |
|---|---|---|---|---|
| Blank group | CD132^{hu/hu} | 5 | Non-modeling | No mAb (PBS) |
| NC group | CD132^{hu/hu} | 6 | Imiquimod modeling | No mAb (PBS) |
| h2D4H4K12 group | CD132^{hu/hu} | 5 | Imiquimod modeling | h2D4H4K12 |

**Table 8 PASI scores of dorsal skin of mice**

| Day | Blank group | | | | | NC group | | | | | | h2D4H4K12 group | | | | |
|---|---|---|---|---|---|---|---|---|---|---|---|---|---|---|---|---|
| 7 | 0 | 0 | 0 | 0 | 0 | 4 | 4 | 4 | 3 | 3 | dead | 1 | 1 | 0 | 2 | 2 |

**Table 9 Body weight statistics of mice in each group**

| | | |
|---|---|---|
| Blank group | NC group | 2D4 group |

Results were shown in FIG. 12: after the disease onset of the psoriasis model mice, the mice in the non-modeling group (Blank group) exhibited rosy dorsal skin without scales, representing normal skin. The mice in the imiquimod modeling untreated group (NC group) exhibited marked erythema and scales on the dorsal skin, and the PASI score of the group was significantly higher than that of the Blank group (p<0.01), while the mice in the h2D4H4K12 treatment group (h2D4H4K12 group) exhibited significantly alleviated erythema and scales compared with the mice in the NC group (p<0.01), and the PASI score of the group was significantly lower than that of the NC group (p<0.01) (Table 8), and the body weight of the mice in the group was also significantly increased (p<0.01) (Table 9). The results indicated that the h2D4H4K12 could significantly alleviate the symptoms of psoriasis.

### Embodiment 9: Evaluation of therapeutic effect of anti-human CD132 antibody h2D4H4K12 on graft-versus-host disease (GVHD)

Experimental procedure: on day 0, B-NDG background mice (Biocytogen, Cat No. 110586) were injected via tail vein with 1×10⁷ human PBMCs. On day 14, peripheral blood was collected from modeled mice, human T/B cells were stained and analyzed by flow cytometry, and cell engraftment was observed. Mice with stable engraftment of the human T/B cells were defined as successful graft-versus-host disease (GVHD) models. The successfully modeled mice were randomly divided into two groups according to cell engraftment and body weight: one group was a NC group (untreated control) and the other group was a h2D4H4K12 group (subcutaneously administered with the h2D4H4K12 at a dose of 10 mg/kg twice per week, until an experimental endpoint. An additional group of unmodeled mice was retained as a Blank group (negative control). The body weight and survival rate of the mice were recorded during treatment. At the experimental endpoint, tissues including liver, spleen and kidney of the mice were harvested and subjected to HE staining to evaluate tissue damage and inflammatory infiltration. Experimental drug administration and treatment protocol for the mice in each group were shown in Table 10.

**Table 10 Experimental drug administration and treatment protocol for mice in each group**

| Group | Strain of recipient | Number of mice per group | Modeling | mAb treatment |
|---|---|---|---|---|
| Blank group | NSG | 6 | Untreated | No mAb (PBS) |
| NC group | NSG | 6 | Tail vein injection with PBMCs | No mAb (PBS) |
| h2D4H4K12 group | NSG | 6 | Tail vein injection with PBMCs | h2D4H4K12 |

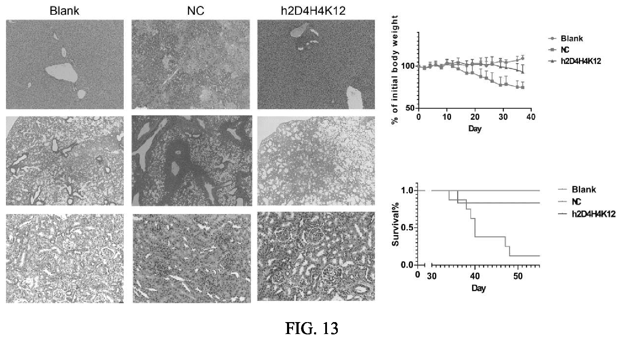

Results were shown in FIG. 13: the mice in the negative control group (Blank group) exhibited continuous body weight gain, good survival status, and no inflammatory cell infiltration or tissue damage in various tissues at the experimental endpoint. However, the mice after GVHD modeling (NC group) exhibited body weight loss over time, poor status and successive death. At the endpoint, a lot of inflammatory infiltration and obvious tissue damage were observed in the liver, lung, kidney and other tissues of the mice in the NC group. However, the administration of the h2D4H4K12 could effectively prevent the development of GVHD in the mice. The mice in the h2D4H4K12 group exhibited less inflammatory cell infiltration in the liver, lung and kidney compared with the mice in the NC group, and most h2D4H4K12-treated mice were protected from body weight loss and death.

In summary, both the in-vitro and in-vivo experiments indicated that the humanized anti-human CD132 antibodies h2D4H4K12 and h5H10H6K4 could inhibit the activities of IL-4, IL-7, IL-9, IL-15 and IL-21, thereby significantly inhibiting the functional activities of the T cells, the B cells and the NK cells in vivo. Abnormal activation of the T cells, the B cells and the NK cells was a key mechanism of pathogenesis of lupus erythematosus, vitiligo and psoriasis. Furthermore, the experiments for the mouse models of lupus erythematosus, vitiligo, psoriasis and GVHD further revealed that both the antibodies could significantly ameliorated the disease phenotype in the mice with lupus erythematosus, and the h2D4H4K12 significantly improved the disease phenotype in the mice with vitiligo, psoriasis and GVHD. Other autoimmune diseases, including rheumatoid arthritis, alopecia areata, inflammatory bowel disease, systemic sclerosis and aplastic anemia, were also closely associated with abnormal activation of the T cells, the B cells or the NK cells. The CD132 served as a key regulatory factor for functions of the T cells, the B cells and the NK cells, and simultaneously, the CD132 served as a common subunit of the above inflammatory cytokines. Therefore, targeting the CD132 was a novel therapeutic direction for patients suffering from various autoimmune diseases.

The above are only the preferred embodiments of the present invention, and it should be pointed out that those of ordinary skills in the art may further make several improvements and decorations without departing from the principle of the present invention, and these improvements and decorations should also be regarded as falling within the scope of protection of the present invention. All the unspecified components in the embodiments can be realized by the prior art.

## Claims

1. An antibody specifically binding to CD132 or an antigen-binding fragment thereof, wherein the antibody or the antigen-binding fragment thereof comprises the following complementarity-determining regions (CDRs):
(a) CDR-H1, CDR-H2 and CDR-H3 contained in a heavy chain variable region (VH) as shown in SEQ ID NO. 1; and CDR-L1, CDR-L2 and CDR-L3 contained in a light chain variable region (VL) as shown in SEQ ID NO. 22;
(b) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 11;
(c) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 16;
(d) the CDR-H1, CDR-H2 and CDR-H3 contained in the heavy chain variable region (VH) as shown in SEQ ID NO. 1; and CDR-L1, CDR-L2 and CDR-L3 contained in a light chain variable region (VL) as shown in SEQ ID NO. 19;
(e) CDR-H1, CDR-H2 and CDR-H3 contained in a heavy chain variable region (VH) as shown in SEQ ID NO. 6; and CDR-L1, CDR-L2 and CDR-L3 contained in a light chain variable region (VL) as shown in SEQ ID NO. 24; and
(f) the CDR-H1, CDR-H2 and CDR-H3 contained in the heavy chain variable region (VH) as shown in SEQ ID NO. 6; and CDR-L1, CDR-L2 and CDR-L3 contained in a light chain variable region (VL) as shown in SEQ ID NO. 29.

2. The antibody or the antigen-binding fragment thereof according to claim 1, wherein the CDRs are defined according to a Kabat, IMGT, Chothia or AbM numbering system.

3. The antibody or the antigen-binding fragment thereof according to claim 1, wherein the VH and/or the VL of the antibody or the antigen-binding fragment thereof comprise framework regions (FRs) derived from a human or mouse immunoglobulin.

4. The antibody or the antigen-binding fragment thereof according to claim 1, wherein the antibody or the antigen-binding fragment thereof binds to human CD132.

5. The antibody or the antigen-binding fragment thereof according to claim 1, wherein the antibody or the antigen-binding fragment thereof comprises:
the following heavy chain variable region (VH) and light chain variable region (VL):
(a) a heavy chain variable region (VH) containing the following 3 CDRs: CDR-H1 having a sequence as shown in SEQ ID NO. 3, CDR-H2 having a sequence as shown in SEQ ID NO. 4 and CDR-H3 having a sequence as shown in SEQ ID NO. 5; and,
a light chain variable region (VL) containing the following 3 CDRs: CDR-L1 having a sequence as shown in SEQ ID NO. 18, CDR-L2 having a sequence as shown in SEQ ID NO. 14 and CDR-L3 having a sequence as shown in SEQ ID NO. 15;
(b) the heavy chain variable region (VH) containing the following 3 CDRs: the CDR-H1 having the sequence as shown in SEQ ID NO. 3, the CDR-H2 having the sequence as shown in SEQ ID NO. 4 and the CDR-H3 having the sequence as shown in SEQ ID NO. 5; and,
a light chain variable region (VL) containing the following 3 CDRs: CDR-L1 having a sequence as shown in SEQ ID NO. 13, the CDR-L2 having the sequence as shown in SEQ ID NO. 14 and the CDR-L3 having the sequence as shown in SEQ ID NO. 15;
(c) the heavy chain variable region (VH) containing the following 3 CDRs: the CDR-H1 having the sequence as shown in SEQ ID NO. 3, the CDR-H2 having the sequence as shown in SEQ ID NO. 4 and the CDR-H3 having the sequence as shown in SEQ ID NO. 5; and,
a light chain variable region (VL) containing the following 3 CDRs: CDR-L1 having a sequence as shown in SEQ ID NO. 21, the CDR-L2 having the sequence as shown in SEQ ID NO. 14 and the CDR-L3 having the sequence as shown in SEQ ID NO. 15;
(d) a heavy chain variable region (VH) containing the following 3 CDRs: CDR-H1 having a sequence as shown in SEQ ID NO. 8, CDR-H2 having a sequence as shown in SEQ ID NO. 9 and CDR-H3 having a sequence as shown in SEQ ID NO. 10; and,
a light chain variable region (VL) containing the following 3 CDRs: CDR-L1 having a sequence as shown in SEQ ID NO. 26, CDR-L2 having a sequence as shown in SEQ ID NO. 27 and CDR-L3 having a sequence as shown in SEQ ID NO. 28; and
(e) the heavy chain variable region (VH) containing the following 3 CDRs: the CDR-H1 having the sequence as shown in SEQ ID NO. 8, the CDR-H2 having the sequence as shown in SEQ ID NO. 9 and the CDR-H3 having the sequence as shown in SEQ ID NO. 10; and,
a light chain variable region (VL) containing the following 3 CDRs: the CDR-L1 having the sequence as shown in SEQ ID NO. 26, the CDR-L2 having the sequence as shown in SEQ ID NO. 27 and CDR-L3 having a sequence as shown in SEQ ID NO. 31.

6. The antibody or the antigen-binding fragment thereof according to claim 1 or 5, wherein the antibody or the antigen-binding fragment thereof comprises the heavy chain variable region (VH) and the light chain variable region (VL) selected from any one of the following groups:
(a) the VH having the sequence as shown in SEQ ID NO. 1 and the VL having the sequence as shown in SEQ ID NO. 22;
(b) the VH having the sequence as shown in SEQ ID NO. 1 and the VL having the sequence as shown in SEQ ID NO. 11;
(c) the VH having the sequence as shown in SEQ ID NO. 1 and the VL having the sequence as shown in SEQ ID NO. 16;
(d) the VH having the sequence as shown in SEQ ID NO. 1 and the VL having the sequence as shown in SEQ ID NO. 19;
(e) the VH having the sequence as shown in SEQ ID NO. 6 and the VL having the sequence as shown in SEQ ID NO. 24; and
(f) the VH having the sequence as shown in SEQ ID NO. 6 and the VL having the sequence as shown in SEQ ID NO. 29.

7. The antibody or the antigen-binding fragment thereof according to claim 1, wherein the antibody or the antigen-binding fragment thereof further comprises:
(a) a heavy chain constant region (CH) of a human immunoglobulin; and
(b) a light chain constant region (CL) of the human immunoglobulin;
wherein, the heavy chain constant region is an IgG heavy chain constant region.

8. The antibody or the antigen-binding fragment thereof according to claim 7, wherein the antibody or the antigen-binding fragment thereof comprises a human IgG4 heavy chain constant region.

9. The antibody or the antigen-binding fragment thereof according to claim 7, wherein the light chain constant region is a kappa chain constant region Ckappa.

10. The antibody or the antigen-binding fragment thereof according to claim 1, wherein the antibody or the antigen-binding fragment thereof is selected from a chimeric antibody, a humanized antibody, a monoclonal antibody, scFv, Fab, Fab', F(ab')2 and Fv fragments, disulfide-linked Fv (dsFv), a diabody, a bispecific antibody and a multispecific antibody.

11. An isolated nucleic acid molecule, encoding the antibody or the antigen-binding fragment thereof according to any one of claims 1 to 10.

12. The isolated nucleic acid molecule according to claim 11, comprising a nucleic acid molecule encoding an antibody heavy chain variable region and a nucleic acid molecule encoding an antibody light chain variable region, wherein,
the nucleic acid molecule encoding the antibody heavy chain variable region is composed of a nucleotide sequence as shown in SEQ ID NO. 2 or 7, and the nucleic acid molecule encoding the antibody light chain variable region is composed of a nucleotide sequence as shown in SEQ ID NO. 23, 12, 17, 20, 25 or 30.

13. A vector, comprising the nucleic acid molecule according to any one of claims 11 to 12.

14. A host cell, comprising the nucleic acid molecule according to any one of claims 11 to 12 or the vector according to claim 13.

15. A preparation method for the antibody or the antigen-binding fragment thereof according to any one of claims 1 to 10, comprising: culturing the host cell according to claim 14 under conditions permitting expression of the antibody or the antigen-binding fragment thereof, and recovering the antibody or the antigen-binding fragment thereof from a culture of the cultured host cell.

16. A pharmaceutical composition, comprising the antibody or the antigen-binding fragment thereof according to any one of claims 1 to 10, or the vector according to claim 13, or the host cell according to claim 14, and a pharmaceutically acceptable carrier and/or excipient.

17. A detection reagent or kit, comprising the antibody or the antigen-binding fragment thereof according to any one of claims 1 to 10, or the vector according to claim 13, or the host cell according to claim 14, or the pharmaceutical composition according to claim 16, and an optional instruction for use.

18. A use of the antibody or the antigen-binding fragment thereof according to any one of claims 1 to 10, or the vector according to claim 13, or the host cell according to claim 14, or the pharmaceutical composition according to claim 16 in manufacture of a drug for treatment and/or prevention of an autoimmune disease, wherein the autoimmune disease is rheumatoid arthritis, systemic lupus erythematosus, vitiligo, psoriasis, alopecia areata, inflammatory bowel disease, systemic sclerosis, graft-versus-host disease (GVHD) or aplastic anemia.

19. A method for prevention and/or treatment of an autoimmune disease, wherein the method comprises administering an effective amount of the antibody or the antigen-binding fragment thereof according to any one of claims 1 to 10, or the vector according to claim 13, or the host cell according to claim 14, or the pharmaceutical composition according to claim 16 to a subject in need thereof.

20. The method according to claim 19, wherein the autoimmune disease is rheumatoid arthritis, systemic lupus erythematosus, vitiligo, psoriasis, alopecia areata, inflammatory bowel disease, systemic sclerosis, graft-versus-host disease (GVHD) or aplastic anemia.

21. A use of the antibody or the antigen-binding fragment thereof according to any one of claims 1 to 10, or the vector according to claim 13, or the host cell according to claim 14, or the pharmaceutical composition according to claim 16 as a drug for treatment and/or prevention of an autoimmune disease, wherein the autoimmune disease is rheumatoid arthritis, systemic lupus erythematosus, vitiligo, psoriasis, alopecia areata, inflammatory bowel disease, systemic sclerosis, graft-versus-host disease (GVHD) or aplastic anemia.

22. A humanized anti-human CD132 monoclonal antibody, wherein the monoclonal antibody is a humanized monoclonal antibody,
wherein, the monoclonal antibody comprises light chain complementarity-determining regions and heavy chain complementarity-determining regions, the light chain complementarity-determining regions comprise LCDR1, LCDR2 and LCDR3, and the heavy chain complementarity-determining regions comprise HCDR1, HCDR2 and HCDR3;
the light chain complementarity-determining regions and the heavy chain complementarity-determining regions are as follows:
amino acid sequences of the LCDR1, LCDR2 and LCDR3 are as shown in SEQ ID NO. 18, SEQ ID NO. 14 and SEQ ID NO. 15 successively; and amino acid sequences of the HCDR1, HCDR2 and HCDR3 are as shown in SEQ ID NO. 3, SEQ ID NO. 4 and SEQ ID NO. 5 successively.

23. The monoclonal antibody according to claim 22, wherein the monoclonal antibody comprises a light chain variable region and a heavy chain variable region, and the light chain variable region and the heavy chain variable region are as follows:
an amino acid sequence of the light chain variable region is as shown in SEQ ID NO. 22; and an amino acid sequence of the heavy chain variable region is as shown in SEQ ID NO. 1.

24. The monoclonal antibody according to claim 22, wherein the monoclonal antibody further comprises constant regions, and the constant regions are a kappa chain constant region Ckappa and a human IgG4 constant region CH1-CH3.

25. A nucleic acid molecule, encoding the monoclonal antibody according to any one of claims 22 to 24.

26. An expression vector, comprising the nucleic acid molecule according to claim 25.

27. A host cell, comprising the expression vector according to claim 26.

28. The host cell according to claim 27, wherein the host cell is a HEK293 cell.

29. A detection reagent or kit, comprising the monoclonal antibody according to any one of claims 22 to 24.

30. A use of the monoclonal antibody according to any one of claims 22 to 24, or the expression vector according to claim 26, or the host cell according to claims 27 to 28 in manufacture of a drug for treatment of an autoimmune disease.

31. The use according to claim 30, wherein the autoimmune disease is rheumatoid arthritis, systemic lupus erythematosus, vitiligo, psoriasis, alopecia areata, inflammatory bowel disease, systemic sclerosis, graft-versus-host disease (GVHD) or aplastic anemia.
